(19) Europäisches Patentamt
European Patent Office
Office européen des brevets

(11) Publication number: 0 463 553 A1

# (12) EUROPEAN PATENT APPLICATION

(21) Application number: 91110030.3

(51) Int. Cl.[5]: C07D 501/36

(22) Date of filing: 19.06.91

(30) Priority: 29.06.90 AT 1393/90
30.07.90 AT 1593/90

(43) Date of publication of application:
02.01.92 Bulletin 92/01

(84) Designated Contracting States:
AT BE CH DE DK ES FR GB GR IT LI LU NL SE

(71) Applicant: BIOCHEMIE GESELLSCHAFT M.B.H.

A-6250 Kundl(AT)

(72) Inventor: Ludescher, Johannes
Breitenbach 109
A-6250 Breitenbach(AT)
Inventor: Wieser, Josef
Schaftenau 76
A-6330 Kufstein(AT)

(74) Representative: Kleine Deters, Johannes et al
c/o Sandoz Technology LTD.,
Patantabteilung
CH-4002 Basel(CH)

## (54) Cephalosporin derivative.

(57) The application relates to a highly pure crystalline modification of cefcanel daloxate hydrochloride (I) and processes for its production.

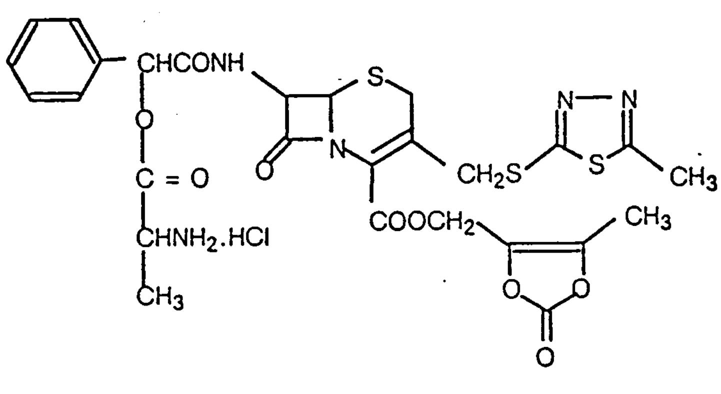

I

EP 0 463 553 A1

The invention relates to a new crystalline modification of cefcanel daloxate hydrochloride of formula I

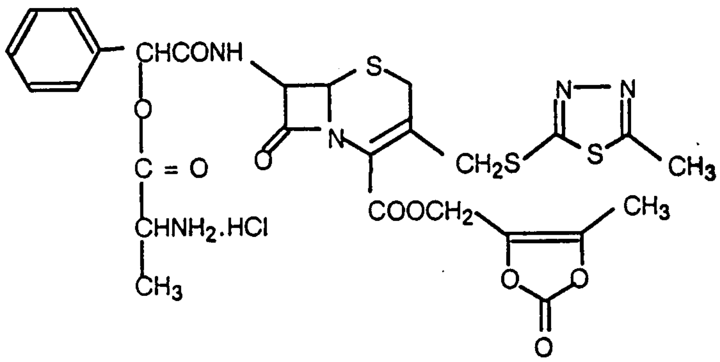

I

and to processes for its production.

Cefcanel daloxate hydrochloride is a bifunctional pro-drug, wherein the 4-carboxyl group is esterified with (5-methyl-2-oxo-1,3-dioxolen-4-yl)methyl and the side chain α-hydroxyl group is esterified with L-alanine. It is a valuable, orally-effective broad spectrum antibiotic, giving high blood levels of the parent drug.

According to conventional processes, cefcanel daloxate hydrochloride is firstly isolated from an organic meduim with an excess of hydrochloric acid, and then recrystallised from alcohol. For example, in EPA 186 706, Example 1, 7-amino-3-(5-methyl-1,3,4-thiadiazol-2-yl)thiomethyl-3-cephem-4-carboxylic acid-(5-methyl-1,3-dioxolen-2-on-4-yl)methylester (AEF) in dichloromethane is reacted with D-O-(L-alanyl)-mandelic acid chloride hydrochloride, and the crude product obtained is recrystallised from ethanol. The hydrochloric acid being released is thereby bound by the resulting product. In further examples, protecting groups on the amino function of the alanyl residue, namely the formyl, 1-methyl-2-ethoxycarbonylvinyl, tert.butoxycarbonyl, benzyloxycarbonyl, trityl and trimethylsilyl protecting group, are removed under acidic conditions and the active substance is isolated with an excess of hydrochloric acid. Thus e.g. in Example 8, the 2,2,2-trichloroethoxycarbonyl protecting group is firstly removed by reduction, cefcanel daloxate is extracted as a free base in dichloromethane, and the product is subsequently produced from methanolic hydrochloric acid and acetone.

In all cases, a product is produced containing more than the stoichiometric quantity of hydrochloric acid. This high content of hydrochloric acid, even on a laboratory scale, can only be removed by drying under protracted and drastic conditions which considerably increases the production time, energy, effort and costs. During this operation, intensive corrosion of the vessel containing the active substance may occur. Moreover the product undergoes discoloration and has inadequate stability.

Crystallisation of the active substance from e.g. ethanol and acetone can only be achieved in satisfactory yield and in a sufficiently good filterable form if hydrochloric acid is added to the system. Without the employment of additional hydrochloric acid on the laboratory scale, under the same conditions, only a yield of 30% was obtained in an ethanol-acetone system. In addition, such products can only be isolated with difficulty and under realistic drying conditions the adherent acetone can hardly be removed. If the active substance is crystallised from pure ethanol without the addition of hydrochloric acid, a suspension which can barely be agitated and filtered is obtained.

It has now been found that the compound of formula I surprisingly forms a stable, pure, highly crystalline modification. These properties render the compound of particular value in pharmaceutical use.

With the new crystalline modification, the above mentioned problems relating to the production of cefcanel daloxate hydrochloride do not arise. Cefcanel daloxate hydrochloride produced by the process according to the invention can be isolated in a simple way, in high yield and with excellent purity, thereby avoiding the use of additional hydrochloric acid. It eliminates the need for corrosion resistant equipment, is less time-consuming, less labour-intensive and environmentally acceptable. It is suitable for industrial scale.

The new crystalline modification may be prepared by crystallising the compound of formula I from acetonitrile. Thus, in another aspect the invention provides a process for the production of cefcanel daloxate hydrochloride which comprises crystallising cefcanel daloxate hydrochloride from acetonitrile. The process may be carried out by crystallising from acetonitrile a cefcanel daloxate hydrochloride which has been produced, if convenient by any method of synthesis. The process may be carried out by dissolving cefcanel

daloxate hydrochloride in acetonitrile at a temperature between about 40° to about 80° C. Precipitation is preferably effected at a temperature of from -20° to +40° C. After precipitation, the product is recovered by filtration and washed and dried in conventional manner.

Alternatively the new crystalline modification may be prepared by adding to a suspension of cefcanel daloxate containing more than the stoichiometric quantity of hydrochloric acid (moist with solvent), which has been produced by any method or purifying operation, as described e.g. in the above-mentioned EPA, in acetonitrile a suitable base. Suitable organic bases are tertiary aliphatic acyclic nitrogen bases with $C_3$- to $C_{12}$-alkyl chains or lipophilic saturated or unsaturated cyclic nitrogen bases. Especially preferred are those bases which form highly soluble hydrochlorides in acetonitrile, e.g. tributylamine or trioctylamine. The process is suitably effected at a temperature from about -40° to about +80° C, preferably at -20° to +25° C.

The new crystalline modification may also be obtained by reacting 7-amino-3-(5-methyl-1,3,4-thiadiazol-2-yl)thiomethyl-3-cephem-4-carboxylic acid-(5-methyl-2-oxo-1,3-dioxolen-4-yl)methylester (AEF) in acetonitrile with D-O-(L-alanyl)mandelic acid chloride hydrochloride, and subsequently adding an equivalent of an organic base. The reaction temperature is suitably from -20° to +60° C, in particular -10° to +25° C. The cephalosporin nucleus can be employed in form of the free base, or can be released in situ in acetonitrile from an acid addition salt, e.g. its dihydrochloride, with one of the bases mentioned below. Suitable organic bases for both purposes are tertiary aliphatic acyclic nitrogen bases with $C_3$- to $C_{12}$-alkyl chains or lipophilic saturated or unsaturated cyclic nitrogen bases. Especially preferred are those bases which form highly soluble hydrochlorides in acetonitrile, e.g. tributylamine or trioctylamine. Surprisingly, acylation can be carried out in acetonitrile without adding a base to neutralize the hydrochloric acid being released during the acylation reaction. In other solvents, e.g. in dichloromethane, the addition of a weak base is necessary, e.g. dimethylacetamide or dimethylformamide, in order to obtain a practically complete reaction. Working up of the mother liquors is simplified as synthesis and isolation of the active substance is carried out exclusively in acetonitrile. (see e.g. dichloromethane: practically unlimited solubility of the monohydrochloride).

The invention provides in one aspect a crystalline modification of cefcanel daloxate hydrochloride showing characteristic intense peaks at interplanar spacings (d) at 11.6, 7.5 and 6.3 Å in the X-ray powder diffraction pattern obtained with nickel filtered copper radiation of $\lambda = 1.54060$ Å.

The above characteristics are based on a diffraction pattern obtained with nickel-filtered copper radiation (Cu $K\alpha_{1+2}$) of wave length $\lambda = 1.54060$ Å using Siemens X-ray diffractometer D-500 with programmable sample changer 40 kV, 20 mA, step-size: 0.01 steps, measuring time: 3 seconds, measuring range: 2 to 40° 2 Theta, 25° C. Internal standard: NBS Silicon SRM 640a. In another aspect the present invention provides a new crystalline modification of cefcanel daloxate hydrochloride showing essentially the following X-ray powder diffraction pattern:

| Interplanar spacing d (Å) | Relative Intensity I |
|---|---|
| 13.514 | 11 |
| 11.617 | 100 |
| 11.106 | 14 |
| 8.312 | 5 |
| 7.508 | 45 |
| 7.117 | 16 |

| Interplanar spacing d (Å) | Relative Intensity I |
|---|---|
| 6.958 | 7 |
| 6.752 | 11 |
| 6.269 | 38 |
| 5.901 | 6 |
| 5.794 | 8 |
| 5.551 | 5 |
| 5.057 | 8 |
| 4.857 | 15 |
| 4.792 | 14 |
| 4.717 | 32 |
| 4.477 | 10 |
| 4.268 | 9 |
| 4.150 | 16 |
| 4.012 | 7 |
| 3.859 | 10 |
| 3.699 | 20 |
| 3.628 | 10 |
| 3.575 | 6 |
| 3.253 | 10 |
| 3.038 | 9 |
| 2.754 | 7 |
| 2.703 | 6 |
| 2.527 | 4 |
| 2.499 | 5 |

It will be appreciated that the above values for "d" and "I" are subject to the deviations usual in the art. For example we have observed deviations in the pattern below d = 5 Å, e.g. the peak at d = 4.717 Å may be split.

The new crystalline modification of cefcanel daloxate hydrochloride is far more stable compared with the cefcanel daloxate hydrochloride produced conventionally by the prior art. This can be shown in an intensified stress test (100° C, 10 hours, effected in a tightly closed vessel) as compared with a sample which has been recrystallised from dichloromethane/ethanol/acetone/hydrochloric acid and dried.

| | |
|---|---|
| New modification: | reduction in content HPLC - 4.3% |
| sample from dichloromethane/EtOH/acetone/HCl: | -10.3% |

The new crystalline modification is useful for the known indications of cefcanel daloxate hydrochloride and has the same order of activity.

In the following Examples, which illustrate the invention more fully but in no way limit its scope, all temperatures are given in degrees Celsius.

**Example 1: Crystalline modification of cefcanel daloxate hydrochloride from acetonitrile**

100 g of dried cefcanel daloxate hydrochloride, crystallised from a mixture of dichloromethane, ethanol, acetone and hydrochloric acid, are dissolved in 2 l of acetonitrile at a water bath temperature of 80°. The hot solution is treated with 10 g of active charcoal and filtered. The product crystallises upon cooling. The crystals are isolated through a suction filter, washed with a little acetonitrile and vacuum dried at room temperature.

| | |
|---|---|
| Yield: | 81 g |
| Content (HPLC): | 98.9% |
| Cl-value (silver titration) | 5.03% |

**Example 2: Crystalline modification of cefcanel daloxate hydrochloride by two-fold recrystallisation from acetonitrile**

200 g of dried cefcanel daloxate hydrochloride, crystallised from a mixture of dichloromethane, ethanol, acetone and hydrochloric acid, are recrystallised in two portions each of 100 g, as described in Example 1. Each 140 g of moist product is recrystallised in 1500 ml of acetonitrile again after treatment with 10 g of charcoal.

| | |
|---|---|
| Content (HPLC): | 99.5% |
| Cl-content (argentometric): | 5.04% |

**Example 3: Crystalline modification of cefcanel daloxate hydrochloride from "cefcanel daloxate dihydrochloride" in acetonitrile**

119.5 g of 7-amino-3-(5-methyl-1,3,4-thiadiazol-2-yl)thiomethyl-3-cephem-4-carboxylic acid-(5-methyl-2-oxo-1,3-dioxolen-4-yl)methylester dihydrochloride (AEC) are added into a mixture of 250 ml of dimethylformamide and 47.5 ml of tributylamine which has been cooled to -10°. After the exothermic neutralisation reaction has died out, 8.8 ml of bistrimethylsilyl acetamide are added to the mixture. After ca. 15 minutes, a clear solution is obtained. 67 g of D-O-(L-alanyl)mandelic acid chloride hydrochloride are subsequently added to the solution at -10° in 8 portions, whereby the temperature rises to a maximum of -5°. The mixture is then stirred for a further 30 minutes at -10°, then heated over a 30 minute-period to 0° and held at this temperature for 60 minutes. Half of the reaction preparation is diluted with 1000 ml of acetone, and 9 ml of concentrated aqueous hydrochloric acid are added. Seed crystals of cefcanel daloxate hydrochloride are subsequently added and after occasional stirring of the resultant acidic crystal suspension at room temperature, the latter is placed in a refrigerator over night. The product is subsequently isolated through a suction filter and washed with acetone in three portions. 30.5 g of the moist product (100.7 g) are dissolved in a mixture of 40 ml of acetone and 8.5 ml of water, and precipitated again by slowly adding a mixture of 800 ml of acetone and 3.4 ml of concentrated hydrochloric acid. After standing the suspension over night in a refrigerator, the product is isolated through a suction filter and washed with acetone.

10 g of this moist product (26.4 g) are suspended in 35 ml of acetonitrile, and the pH value is adjusted to 4.2 by stirring in a total of 2.15 ml of tributylamine. A coarse crystal suspension is thus obtained. This is stirred for 2 hours at room temperature and subsequently placed in the refrigerator over night. The cefcanel daloxate hydrochloride is subsequently isolated through a suction filter, washed with acetonitrile and vacuum dried.

| | |
|---|---|
| Yield: | 6.1 g (76.2% of theory based on AEC) |
| Content (HPLC): | 99.2% |

**Example 4: Synthesis of cefcanel daloxate hydrochloride in acetonitrile without the addition of base for acylation**

46.7 g of 7-amino-3-(5-methyl-1,3,4-thiadiazol-2-yl)thiomethyl-3-cephem-4-carboxylic acid-(5-methyl-2-oxo-1,3-dioxolen-4-yl)methylester (AEF) are introduced to 625 ml of acetonitrile and stirred until a solution is formed. The solution is cooled to -10° and treated with 33.5 g of D-O-(L-alanyl)mandelic acid chloride hydrochloride in 8 portions over a 30 minute-period. During this addition, the temperature is maintained below -5°. The suspension is subsequently maintained for 30 minutes in a temperature range between -10 and -5°, and is then heated to 0° over a 30 minute-period. After 30 minutes at this temperature, the suspension is heated at 30 minute intervals to 10, 15 and then 20°. 23.8 ml of tributylamine are subsequently added in drops to the suspension a ca. 15 minute-period. Upon the addition of a further 4.8 ml of tributylamine, a macroscopically visible change of the crystal suspension is observed. The suspension is stirred for ca. 30 minutes, and after standing over night, the title compound is isolated through a suction filter, washed with 75 ml of cold acetonitrile and dried in vacuum.

| | |
|---|---|
| Yield: | 60.6 g (86.8% of theory) |
| Content (HPLC): | 99.7% |

## Claims

1. A crystalline modification of cefcanel daloxate hydrochloride showing characteristic intense peaks at interplanar spacings (d) at 11.6, 7.5 and 6.3 Å in the X-ray powder diffraction pattern obtained with nickel filtered copper radiation of λ = 1.54060 Å.

2. Crystalline modification of cefcanel daloxate hydrochloride showing essentially the following X-ray powder diffraction pattern obtained with nickel-filtered copper radiation of λ = 1.54060 Å:

| Interplanar spacing d (Å) | Relative Intensity I |
|---|---|
| 13.514 | 11 |
| 11.617 | 100 |
| 11.106 | 14 |
| 8.312 | 5 |
| 7.508 | 45 |
| 7.117 | 16 |
| 6.958 | 7 |
| 6.752 | 11 |
| 6.269 | 38 |
| 5.901 | 6 |
| 5.794 | 8 |
| 5.551 | 5 |
| 5.057 | 8 |
| 4.857 | 15 |
| 4.792 | 14 |
| 4.717 | 32 |

| Interplanar spacing d (Å) | Relative Intensity I |
|---|---|
| 4.477 | 10 |
| 4.268 | 9 |
| 4.150 | 16 |
| 4.012 | 7 |
| 3.859 | 10 |
| 3.699 | 20 |
| 3.628 | 10 |
| 3.575 | 6 |
| 3.253 | 10 |
| 3.038 | 9 |
| 2.754 | 7 |
| 2.703 | 6 |
| 2.527 | 4 |
| 2.499 | 5 |

**3.** A process for the production of the crystalline modification of cefcanel daloxate hydrochloride as defined in claim 1 which comprises crystallising cefcanel daloxate hydrochloride from acetonitrile.

**4.** A process according to claim 3 for the production of the crystalline modification cefcanel daloxate hydrochloride as defined in claim 1 which comprises adding an organic base to a suspension of cefcanel-daloxate containing more than the stoichiometric quantity of hydrochloric acid the suspension being in acetonitrile.

**5.** A process according to claim 3 which comprises reacting 7-amino-3-(5-methyl-1,3,4-thiadiazol-2-yl)-thiomethyl-3-cephem-4-carboxylic acid(5-methyl-2-oxo-1,3-dioxolen-4-yl)methylester, or its dihydrochloride after adding a stoichiometric quantity of an organic base in acetonitrile with D-O-(L-alanyl)-mandelic acid chloride hydrochloride and subsequently adding an organic base.

**6.** A process according to claim 4 or 5, wherein the organic base employed is a tertiary aliphatic acyclic nitrogen base with $C_3$- to $C_{12}$-alkyl chains or a lipophilic saturated or unsaturated cyclic nitrogen base.

**7.** A process according to claim 4, 5 or 6, wherein tributylamine is employed as the base.

**8.** A process according to claim 5, wherein the acylation reaction is carried out without a base scavenger.

European Patent Office

# EUROPEAN SEARCH REPORT

Application Number

**EP 91 11 0030**

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| A,D | EP-A-0 186 706 (KYOTO PHARMACEUTICAL INDUSTRIES, LTD.) * page 3, line 1 - page 4, line 18 * * claim * | 1,5 | C 07 D 501/36 |
| A | EP-A-0 153 418 (KYOTO PHARMACEUTICAL INDUSTRIES, LTD.) * page 1, line 1 - page 3, line 5; claims 1, 9 * | 1,5 | |
| A | EP-A-0 180 372 (UPJOHN CO.) * page 1, line 19 - page 2, line 1; claims 1, 3, 4 * | 1-4 | |

TECHNICAL FIELDS SEARCHED (Int. Cl.5)

C 07 D 501/00

The present search report has been drawn up for all claims

| Place of search | Date of completion of search | Examiner |
|---|---|---|
| Berlin | 01 October 91 | HASS C V F |

CATEGORY OF CITED DOCUMENTS

X : particularly relevant if taken alone
Y : particularly relevant if combined with another document of the same catagory
A : technological background
O : non-written disclosure
P : intermediate document
T : theory or principle underlying the invention
E : earlier patent document, but published on, or after the filing date
D : document cited in the application
L : document cited for other reasons

& : member of the same patent family, corresponding document